# EUROPEAN PATENT SPECIFICATION

(11) **EP 4 363 396 B1**
(45) Date of publication and mention of the grant of the patent: **15.04.2026**
(21) Application number: 22738642.2
(22) Date of filing: 29.06.2022
(51) Int. Cl.: C07C 209/36, C07C 211/46

(54) **PROCESS FOR PRODUCING ANILINE**
VERFAHREN ZUR HERSTELLUNG VON ANILIN
PROCÉDÉ DE PRODUCTION D'ANILINE

(30) Priority: 30.06.2021 EP 21182858
(43) Date of publication of application: 08.05.2024
(73) Proprietor: BASF SE, 67056 Ludwigshafen am Rhein (DE)
(72) Inventor: DE WINNE, Hendrik, 2040 Antwerpen (BE); BLANKERS, Bart, 2040 Antwerpen (BE)
(74) Representative: BASF IP Association
(86) International application number: PCT/EP2022/067884
(87) International publication number: WO 2023/275137

(56) References cited:
- EP-A1- 0 458 006
- US-A- 3 136 818
- US-A- 3 270 057
- US-A- 5 962 365

## Description

The invention relates to a process for producing aniline by catalytic hydrogenation of nitroben-zene in which hydrogen and nitrobenzene are reacted in the presence of a catalyst in a hydrogenation reactor to form a crude reaction product and the crude reaction product is worked up to obtain purified aniline.

The hydrogenation of nitrobenzene to obtain aniline can be carried out in the gas phase or in the liquid phase. If the hydrogenation is carried out in the gas phase, the catalyst, which is used in the hydrogenation reaction, may either be present in a fixed bed as described for example in US 4,740,621 or EP-A 748 790, or in a fluidized bed as described in US 3,136,818. A hydrogenation reaction of nitrobenzene to obtain aniline in the liquid phase is disclosed for example in US 4,415,754 or US 3,270,057.

EP 0,458,006 further describes a process for the production of aniline by catalytic hydrogenation of nitrobenzene.

The crude reaction product obtained by the hydrogenation of nitrobenzene comprises aniline as product, hydrogen, water and further by-products, for example phenols, aminophenols and phenazines. Depending on the type of reaction, the crude reaction product further may contain nitrobenzene which did not react.

If the reaction is carried out in the gas phase, usually the crude reaction product is condensed, wherein the condensation may be carried out in one step or more than one step and with or without heat integration.

The crude reaction product obtained in the liquid phase reaction or the condensed crude reaction product obtained after condensation of the crude reaction product of the gas phase reaction is followed by a gas/liquid separation in which more than 95% of the reaction water and the aniline are condensed and the gas phase containing hydrogen is removed.

The thus obtained liquid phase is subject to a liquid/liquid separation which can be carried out in one or more stages. By the liquid/liquid separation an organic phase comprising aniline and an aqueous phase are obtained. The organic phase is further worked-up by distillation to remove low boilers, particularly remaining water, and high boilers to obtain pure aniline as product. As the stream containing high boilers which is removed from the process and usually incinerated still contains aniline, WO-A 2014/057053 describes a process for working-up the crude aniline in which the loss of aniline is further reduced.

However, it is a problem of all processes for producing aniline that the catalyst activity decreases over time and that by coming close to the end of run-time or end of life-time of the catalyst, the amount of nitrobenzene which did not react increases and, thus, the nitrobenzene breaks through which results in a loss of quality of the aniline and a reduction of conversion and yield.

Therefore, it is an object to provide a process for producing aniline by catalytic hydrogenation of nitrobenzene where deactivation of the catalyst can be timely recognized to avoid a quality loss of aniline due to break-through of nitrobenzene and to have enough time for planning catalyst regeneration or exchange with only short interruption of the process.

This object is achieved by a process for producing aniline by catalytic hydrogenation of nitrobenzene comprising:
(a) reacting hydrogen and nitrobenzene in the presence of a catalyst in a hydrogenation reactor, forming a crude reaction product;
(b) working up the crude reaction product to obtain pure aniline,
wherein the composition of the crude reaction product is analysed continuously or at regular time intervals before starting working-up in (b), for determining the proportion of by-products, wherein, in the event of an increase in the amount of by-products, measures are taken to prevent nitrobenzene from breaking through in the hydrogenation reactor, wherein the measures which are taken to prevent nitrobenzene from breaking through in the hydrogenation reactor comprise at least one of those defined in present claim 1.

Surprisingly it has shown that the amount of by-products in the crude reaction product starts to increase some days before deactivation of the catalyst can be observed by analysis of the pure aniline, which is the usual process. Most of these by-products that allow the early observation of the deactivation of the catalyst are not found in the pure aniline product because they are removed from the crude reaction product in the usual purification steps to produce pure aniline.

Due to the lifetime of the catalyst which, depending on the catalyst system can be anywhere between a few weeks and several months, the regular time intervals for analysing the crude reaction product, if the crude reaction product is analysed in regular time intervals and not continuously, may be up to 24 h, preferably in a range between 18 h and 10 h and particularly between 10 h and 6 h. A regular time interval of 24 h means that only once a day the composition of the crude reaction product is analysed during normal operation of the process.

By the early detection of upcoming deactivation of the catalyst, it is possible to take several measures to continue producing pure aniline still having the required properties. Particularly, process parameters can be altered to further continue pure aniline with a required low level of nitrobenzene contained in the pure aniline until it is possible to regenerate or change the catalyst with only a short interruption in the production process. Taking measures at such an early stage has the additional advantage that the quality of the pure aniline produced in the hydrogenation reactor has the same quality all time and even shortly to the end of runtime or end of lifetime of the catalyst no pure aniline is produced which does not comply with the required properties to be used in most applications in which aniline is used, for example in the production of methylene diphenyl diisocyanate (MDI).

On the other hand, late detection by analysis of the pure aniline as known from the usual processes leaves little time to stop the production process to prevent that pure aniline is produced which does not comply with the required properties like nitrobenzene concentration. This can lead to an unscheduled interruption of the production in the downstream production plants, using aniline as feed material, due to lack of supply.

If the production process is not stopped in time, further deactivation of the catalyst significantly increases the concentration of nitrobenzene in the pure aniline that is produced causing the pure aniline to no longer comply with the required properties. This pure aniline would require expensive and energy intensive treatment to remove the nitrobenzene to below the requested concentration. Additionally, the customer could demand financial compensation in case they received the pure aniline with a concentration of nitrobenzene above the requested concentration.

The measures which are taken to prevent nitrobenzene from breaking through in the hydrogenation reactor particularly comprise at least one of:
- scheduling regeneration or replacement of the catalyst and foresee sufficient inventory of pure aniline to compensate the downtime of the plant during said regeneration or replacement;
- decreasing plant load to postpone catalyst deactivation;
- temporarily increase the time intervals for analysing the composition of the crude reaction product;
- increasing the temperature of the reaction to increase reactivity and to postpone the catalyst deactivation.

In this context "plant load" refers to the nitrobenzene feed rate to the hydrogenation reactor and as such the production rate of the plant. As such, a decreasing plant load refers to a decreasing feed rate of nitrobenzene to the hydrogenation reactor.

As by the inventive process the upcoming deactivation is detected up to several days before nitrobenzene starts to break through, it is possible to schedule regeneration or replacement of the catalyst in time and particularly have enough time left to foresee a sufficient inventory of pure aniline. To foresee the sufficient inventory of pure aniline, it is for example possible to increase the production rate of another aniline plant on the same site, import aniline from other production sites or purchase aniline from other manufacturers or reduce sales of aniline leading up to, or during the catalyst regeneration or replacement.

If it is not possible to regenerate or replace the catalyst in the remaining lifetime which is determined by the analysis of the by-products, it is preferred to decrease the plant load. By decreasing the plant load, the catalyst deactivation is postponed and there is additional time gained until the catalyst needs to be regenerated or replaced.

As an alternative or an additional measure for postponing the catalyst deactivation, it is possible to temporarily increase the temperature of the reaction. By increasing the temperature, also the reactivity is increased and due to the increased reactivity, the catalyst deactivation is postponed. For increasing the reactivity and, thereby, postponing the catalyst deactivation, it is preferred to increase the reactor temperature by at least 2 °C, particularly by more than 5 °C. The temperature increase thereby depends on the reaction type, the reactor type, reactor operation, particularly whether isothermally or adiabatically and overall plant setup.

Further, as it has shown that the increase of by-products changes from a linear increase to an exponential increase the closer the catalyst comes to being deactivated, it is further preferred to temporarily decrease the time intervals and thus increase the frequency for analysing the composition of the crude reaction product. By this decrease of the time intervals it is possible to stop the reaction at a closer point to the final deactivation of the catalyst and, thus, to avoid nitrobenzene to break through but to continue the reaction as long as possible and to regenerate or replace the catalyst only very short before its final deactivation. Therefore, independently of which measures are taken for postponing the catalyst deactivation or scheduling the regeneration or replacement of the catalyst, it is particularly preferred to additionally temporarily decrease the time intervals for analysing the composition of the crude reaction product in case the analysis of the composition of the crude reaction product is not carried out continuously.

The hydrogenation reaction for producing aniline in the presence of a catalyst can be any type of reaction of hydrogen and nitrobenzene, particularly mononitrobenzene (MNB), which is known to a skilled person. The reaction of hydrogen and nitrobenzene may be carried out for example in the liquid phase or in the gas phase.

If the reaction is carried out in the liquid phase, usually liquid nitrobenzene is fed into a hydrogenation reactor where it is brought into contact with hydrogen. The hydrogenation reactor contains the catalyst which is in the shape of small particles and forms a slurry with the liquid nitrobenzene. The reaction may be carried out at a temperature in the range from 100 to 300°C. Due to the temperature in the hydrogenation reactor, the crude reaction product may be in the vapour phase which is withdrawn from the hydrogenation reactor and is condensed subsequently. As besides the aniline also water is formed, it is necessary to separate the aniline from the water, for this purpose, a phase separation can be carried out in which the aqueous phase containing the water is separated from the organic phase which contains the aniline and corresponds to the crude reaction product.

If the reaction is carried out in the gas phase, in difference to carrying out the reaction in the liquid phase, the nitrobenzene is evaporated before being fed into the hydrogenation reactor. The catalyst in the gas phase reaction may either be in the form of a fluidized bed or in the form of a fixed bed. Independently of being in the form of a fluidized bed or a fixed bed, it is preferred to mix the hydrogen and the evaporated nitrobenzene before feeding into the hydrogenation reactor. Mixing the hydrogen and the evaporated nitrobenzene can be carried out in any manner known to a skilled person. If the catalyst is a fluidized bed, the catalyst is fluidized by the gaseous reactant mixture containing hydrogen and nitrobenzene. If the catalyst is a fixed bed, the reactant mixture is fed into the catalyst bed in which the hydrogen and the nitrobenzene react. As the reaction is exothermic, also the crude reaction product is in the gas phase which is withdrawn from the reactor and condensed. Condensation, thereby, can be carried out in only one step or in two or more steps.

To achieve pure aniline, it is necessary to work-up the crude reaction product for removing by-products which are produced in the reaction and to remove further impurities which may be contained in the reactants and thus may be introduced with the reactants.

Independently of being carried out in the liquid phase or in the gas phase, by the catalytic reaction of hydrogen and nitrobenzene, besides aniline, water is produced. Thus, it is necessary to separate the water from the aniline. For removing the water, the crude reaction product, which is withdrawn from the reactor preferably is at least partially condensed. As the boiling point of aniline is higher than the boiling of water, the aniline starts to condensate first and the water, which is still in the gas phase can be removed. As usually only a part of the aniline is condensed in the first step, the vapour removed from the first condensation still contains product. For this purpose, further condensation steps are provided and the condensation is carried out in two or more steps, where in each step aniline is condensed from the vapour and the gaseous water which contains less aniline after each condensation step is fed into a following condensation step or after being withdrawn from the last condensation step is removed from the process.

As in each condensation step besides aniline also some water may condense and further high boiling by-products and impurities, it is necessary to further work-up the condensed phase. If condensation is carried out in more than one condensation step, the condensed phases of all steps are combined and fed into the working-up step (b). Working-up the crude reaction product is carried out according to the well-known processes, particularly by distillation in one or more steps to remove remaining low-boiling components and the high-boiling by-products and impurities.

In the context of the present invention, "high-boiling" means that the boiling point of the respective components is higher than the boiling point of aniline and "low-boiling" means that the boiling point of the respective components is below the boiling point of aniline.

If the crude reaction product is withdrawn from the reactor in the gas phase, it is possible to analyse the composition of the gaseous crude reaction product. However, preferably, the composition of liquid crude reaction product, i.e. the at least partly condensed crude reaction product, is analysed. For this purpose, if condensation is carried out in only one step, the condensed crude reaction product withdrawn from the condensation is analysed. If the condensation is carried out in at least two condensation step, it is possible to analyse the composition of the crude reaction product which is obtained in any of the condensation steps. If the condensed phases of all condensation steps are combined, it is also possible to analyse the composition of the combined crude reaction product.

For detecting the catalyst deactivation, the crude reaction product is analysed for those by-products which are a result of side reactions in the hydrogenation reactor that increasingly take place due to catalyst deactivation. These by-products particularly are high boiling components.

Particularly, the by-product, the proportion of which is analysed, is at least one of aminophenols, n-phenyl-1,2-benzenediamine, n-phenyl-1,4-benzenediamine, phenazine, phenol, nitrobenzene, and unidentifiable high boiling components. "Unidentifiable high boiling components" are those components which give a signal in the used analysing method but where the signal cannot be connected to a specific component so that a component is detected but it is not possible to identify this specific component.

As high-boiling by-products and impurities start to condense first, the amount of these high-boiling by-products is maximally in the condensed phase of the condensation step. Therefore, it is particularly preferred, to analyse the partly condensed crude reaction product obtained in the first condensation step.

During normal operation when the catalyst has a normal activity, the concentration of by-products is stable. An upcoming deactivation of the catalyst is indicated by an increase in the concentration of the by-products of more than 10 % compared to the stable concentration. More preferred, the upcoming deactivation of the catalyst is indicated by an increase in the concentration of the by-products of more than 20 % and particularly of more than 30 % compared to the stable concentration. Additionally, the upcoming deactivation of the catalyst often is indicated by an additional combination of the increase of the amount of by-products with a transition from a linear increase to an exponential increase.

The increase in the concentration of the by-products and the transition from linear increase to an exponential increase usually can be observed some days before the catalyst completely deactivates and nitrobenzene starts to break through. Therefore, it is particularly preferred to take the measures to prevent nitrobenzene from breaking through in the hydrogenation reactor when the increase of the amount of by-products in the crude reaction product shows a transition from a linear to an exponential increase.

Analysis of the composition of the crude reaction product can be done by any analysing method which allows for analysing the composition of a stream known to a skilled person. Suitably analysing methods particularly are chromatographic or spectroscopic techniques. Chromatographic techniques for example include gas chromatography (GC) or high performance liquid chromatography (HPLC) with gas chromatography being particularly preferred. Spectroscopic techniques which can be used for analysing the composition of the crude reaction product particularly comprise for example infrared spectroscopy (IR), near infrared spectroscopy (NIR), nuclear magnetic resonance spectroscopy (NMR) or Raman-spectroscopy. Preferred spectroscopic techniques are near infrared spectroscopy or Raman-spectroscopy.

Independently of being carried out continuously or at regular time intervals, the analysis of the crude reaction product can be carried out by online analysis processes. If the analysis of the crude reaction product is carried out at regular time intervals, it is also possible that for analysing the composition of the crude reaction product a sample is taken and the sample is analysed.

If an online analysis process is used for analysing composition of the crude reaction product, it is necessary to use an analysing method which can be operated continuously. For this purpose, for example gas chromatography is appropriate.

If samples of the crude reaction product are taken and the samples are analysed, it is particularly preferred to use gas chromatography for analysing the crude reaction product regarding its composition.

An embodiment of the invention is shown in the figure and explained in more detail in the following description.

In the figures:
- Figure 1: shows schematically a process for producing aniline in the gas phase;
- Figures 2 to 5: show the concentration gradients of several by-products depending on the time.

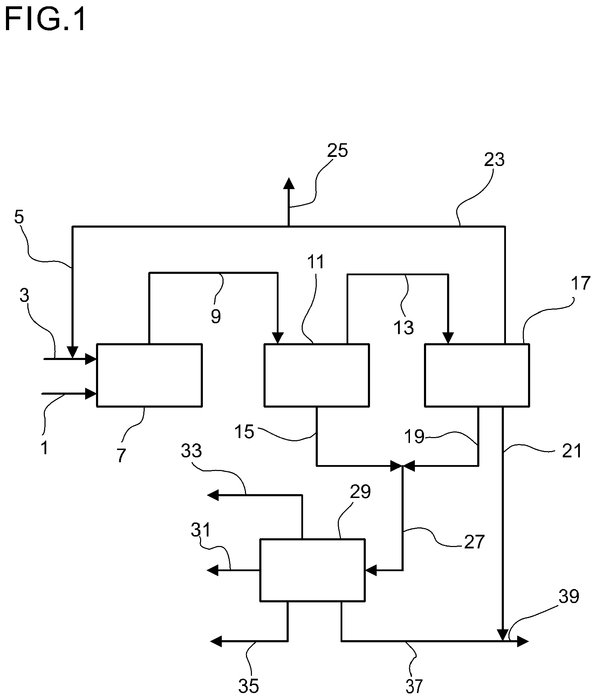

Figure 1 shows a process for producing aniline in the gas phase.

For producing aniline, nitrobenzene 1 and hydrogen 3 and preferably recycle hydrogen 5 are fed into a hydrogenation reactor 7. In the hydrogenation reactor, a crude reaction product 9 is formed. The crude reaction product 9 comprises aniline, water, remainders of nitrobenzene and hydrogen which did not react and by-products which also are formed in the reaction. Typical by-products for example are phenol, phenazine, n-phenyl-1,2-benzenediamine and n-phenyl-1,4-benzenediamine.

The crude reaction product 9 is transferred into a first condensation 11. In the first condensation 11, the crude reaction product 9 is cooled to a temperature below the boiling point of aniline and above the boiling point of water, such that the aniline condenses and the water remains in the gas phase. From the first condensation 11, gaseous components 13 containing water, aniline and low boilers, and a liquid stream 15, containing crude aniline and high boiling by-products are withdrawn.

The gaseous components 13 which still contain aniline, are transferred into a second condensation 17. The second condensation 17 is carried out in such a way, that crude aniline can be withdrawn as a first liquid stream 19 and waste water as a second liquid stream 21. Further, gaseous components, particularly hydrogen which did not react are withdrawn from the second condensation 17 as gas stream 23.

The hydrogen containing gas stream may be recycled at least partly into the hydrogenation reactor 7 as recycle hydrogen 5. If the hydrogen is not recycled or only a part of the hydrogen is recycled, the non-recycled part is removed as hydrogen off-gas 25. As the gas stream 23 may contain further gaseous components besides hydrogen, it is preferred to recycle only a part of the gas stream 23 into the hydrogenation reactor 7 and to remove a part of the gas stream 23 as hydrogen off-gas 25, to avoid accumulation of the further gaseous components contained in the gas stream 23.

The first liquid stream 19 containing the crude aniline obtained in the second condensation and the liquid stream 15 containing the crude aniline obtained in the first condensation preferably are combined into a combined stream 27. The combined stream 27 then is fed into a crude aniline purification 29. In the crude aniline purification 29, which may be carried out in one or more distillation steps, pure aniline 31, light boilers 33 and high boilers 35 are obtained.

Further, it is preferred to separate the water from the light boilers 33 and to remove the water 37 separately. The water 37 obtained in the crude aniline purification 29 and the water obtained as second liquid stream 21 in the second condensation 17 preferably are combined and removed from the process as waste water 39. This waste water 39 preferably is transferred into a water purification plant for further treatment.

According to the invention, it is preferred to analyse the composition of the crude aniline which was obtained as liquid stream 15 in the first condensation 11. However, besides analysis of the liquid stream 15 it is also possible to analyse the combined stream 27, which contains the crude aniline obtained in the first condensation 11 and the second condensation 17, or the first liquid stream 19 containing the crude aniline obtained in the second condensation 17. However, analysing the first liquid stream 19 obtained in the second condensation is least preferred.

### Examples:

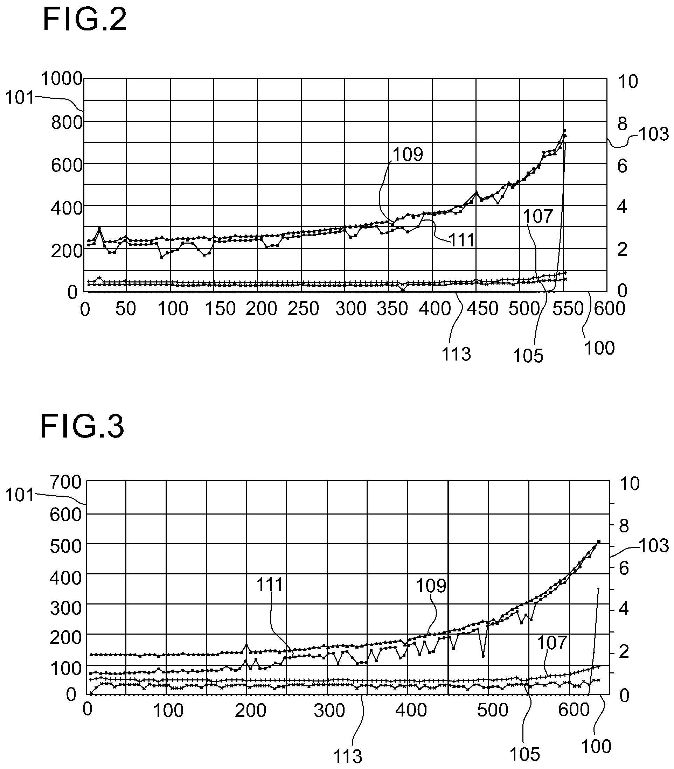

In figures 2 to 5, showing the results of the examples, the abscissa 100 shows the time in hours, the ordinate 101 on the left the concentration of by-products in mg/kg, and the ordinate 103 on the right the concentration of nitrobenzene in mg/kg. Further, the curves in the graphs are assigned as follows:
- 105: phenol
- 107: phenazine
- 109: n-phenyl-1,2-benzenediamine
- 111: n-phenyl-1,4-benzenediamine
- 113: nitrobenzene.

### Example 1

Aniline is produced in a plant as shown in figure 1. During the production process the concentrations of several by-products are observed. This is shown for the last 550 h before catalyst deactivation in figure 2.

Until timestamp 150 to 200 h in the graph, the concentration of n-phenyl-1,2-benzenediamine, n-phenyl-1,4-benzenediamine and phenazine is stable, showing that the catalyst is still operating at normal activity.

At timestamp 300 h, the concentration in n-phenyl-1,2-benzenediamine, n-phenyl-1,4-benzenediamine and phenazine has increased to a value which is more than 10 % higher than the stable values. By the increase, a decline in catalyst activity and an upcoming breakthrough of nitrobenzene is indicated.

At timestamp 450 h, the concentration in n-phenyl-1,2-benzenediamine, n-phenyl-1,4-benzenediamine and phenazine has increased to a value which is more than 20 % higher than the stable values. The increase in concentration also changes from linear to exponential. Both indicate a further decline in catalyst activity and an upcoming nitrobenzene breakthrough within the next 4 to 5 days. Regeneration of the catalyst was scheduled and sufficient inventory of pure aniline was foreseen to continue supply to customers during the catalyst regeneration. If no action had been taken, the downstream production plants, using aniline as feed material, would have had to be shut down due to lack of supply during the regeneration of the catalyst. Further, the sampling frequency can be increased to monitor breakthrough more regularly.

At timestamp 546 h breakthrough of nitrobenzene is measured in the crude reaction product and the production stopped as scheduled before any nitrobenzene was measured in the pure aniline.

### Example 2

A further production process was carried out in the plant as shown in figure 1. The concentrations of the by-products which were observed are shown in time-dependency in the graph of figure 3.

Until timestamp 150 to 200 h in the graph, the concentration of n-phenyl-1,2-benzenediamine, n-phenyl-1,4-benzenediamine and phenazine is stable, showing that the catalyst is still operating at normal activity.

At timestamp 350 h, the concentration in n-phenyl-1,2-benzenediamine, n-phenyl-1,4-benzenediamine and phenazine has increased to a value which is more than 10 % higher than the stable values. By the increase, a decline in catalyst activity and an upcoming breakthrough of nitrobenzene is indicated.

At timestamp 500 h, the concentration in n-phenyl-1,2-benzenediamine, n-phenyl-1,4-benzenediamine and phenazine has increased to a value which is more than 30 % higher than the stable values. The increase in concentration also changes from linear to exponential. Both indicate a further decline in catalyst activity and an upcoming nitrobenzene breakthrough within the next 4 to 5 days. Regeneration of the catalyst was scheduled and sufficient inventory of pure aniline was foreseen to continue supply to customers during the catalyst regeneration. If no action had been taken, the downstream production plants, using aniline as feed material, would have had to be shut down due to lack of supply during the regeneration of the catalyst. Further, the sampling frequency can be increased to monitor breakthrough more regularly.

Besides scheduling the catalyst regeneration, it is also possible to decrease the plant load to continue production to a scheduled shutdown a few days later. Alternatively, reaction temperature can be increased to postpone breakthrough of nitrobenzene. This, however, also increases the deactivation rate of the catalyst due to the increased rate at which heavy deposits ("cokes") are built on the catalyst at higher temperatures and which additionally must be removed during catalyst regeneration.

### Example 3

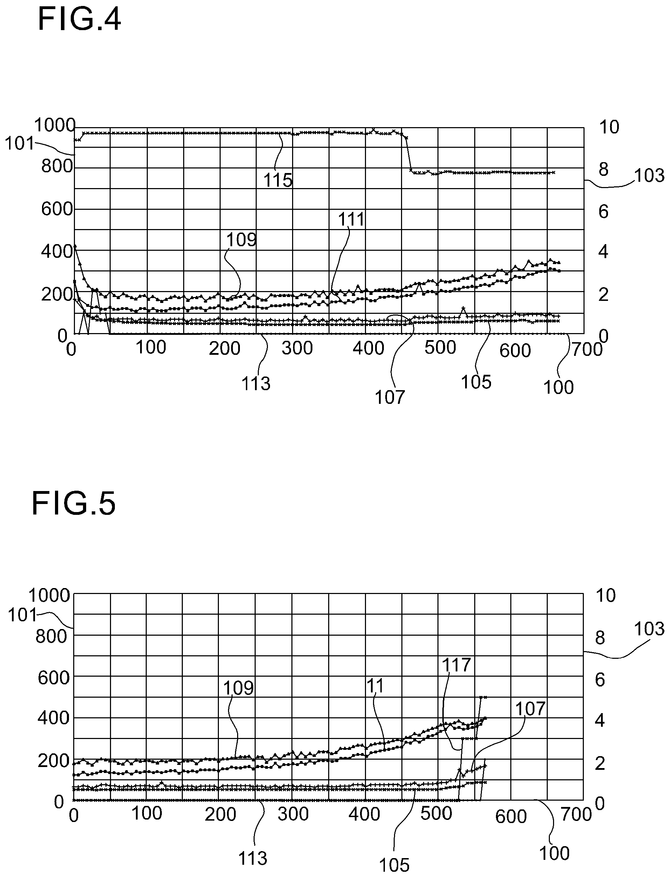

A further production process was carried out in the plant as shown in figure 1. The concentrations of the by-products which were observed are shown in time-dependency in the graph of figure 4.

Until timestamp 250 h in the graph, the concentration of n-phenyl-1,2-benzenediamine, n-phenyl-1,4-benzenediamine and phenazine is stable, showing that the catalyst is still operating at normal activity.

At timestamp 380 h, the concentration in n-phenyl-1,2-benzenediamine, n-phenyl-1,4-benzenediamine and phenazine has increased to a value which is more than 10 % higher than the stable values. By the increase, a decline in catalyst activity and an upcoming breakthrough of nitrobenzene is indicated.

At timestamp 450 h, the concentration in n-phenyl-1,2-benzenediamine, n-phenyl-1,4-benzenediamine and phenazine has increased to a value which is almost 20 % higher than the stable values. The load of the plant is decreased from ±100% to ±80% to continue production to a scheduled shutdown a few days later at timestamp 660 h. At that timestamp, the increase in concentration is still linear. The ordinate 103 on the right in figure 4 additionally indicates the plant load in [%/10]. The graph of the plant load is assigned reference number 115.

Alternatively, reaction temperature can be increased to postpone breakthrough of nitrobenzene. This, however, also increases the deactivation rate of the catalyst due to the increased rate at which heavy deposits ("cokes") are built on the catalyst at higher temperatures and which additionally must be removed during catalyst regeneration.

### Example 4

A further production process was carried out in the plant as shown in figure 1. The concentrations of the by-products which were observed are shown in time-dependency in the graph of figure 5.

Until timestamp 200 h in the graph, the concentration of n-phenyl-1,2-benzenediamine, n-phenyl-1,4-benzenediamine and phenazine is stable, showing that the catalyst is still operating at normal activity.

At timestamp 300 h, the concentration in n-phenyl-1,2-benzenediamine, n-phenyl-1,4-benzenediamine and phenazine has increased to a value which is more than 10 % higher than the stable values. By the increase, a decline in catalyst activity and an upcoming breakthrough of nitrobenzene is indicated.

At timestamp 400 h, the concentration in n-phenyl-1,2-benzenediamine, n-phenyl-1,4-benzenediamine and phenazine has increased to a value which is more than 30 % higher than the stable values. The increase in concentration also changes from linear to slightly exponential. Both indicate a further decline in catalyst activity and an upcoming nitrobenzene breakthrough within the next 4 to 5 days. Regeneration of the catalyst can be scheduled and sufficient inventory of pure aniline can be foreseen to continue supply to customers during the catalyst regeneration. Further, the sampling frequency can be increased to monitor breakthrough more regularly.

At timestamp 528 h the reactor temperature is increased by 3°C, consequently the concentration in n-phenyl-1,2-benzenediamine, n-phenyl-1,4-benzenediamine and phenazine decreases, indicating that the breakthrough of nitrobenzene is postponed. This, however, also increases the deactivation rate of the catalyst due to the increased rate at which heavy deposits ("cokes") are built on the catalyst at higher temperatures and which additionally must be removed during catalyst regeneration.

The reactor temperature increase is shown on the ordinate 103 on the right and the graph of the temperature increase is assigned reference number 117.

## Claims

1. A process for producing aniline by catalytic hydrogenation of nitrobenzene comprising:
(a) reacting hydrogen and nitrobenzene in the presence of a catalyst in a hydrogenation reactor, forming a crude reaction product;
(b) working up the crude reaction product to obtain pure aniline,
wherein the composition of the crude reaction product is analysed continuously or at regular time intervals before starting working-up in (b), for determining the proportion of by-products, wherein, in the event of an increase in the amount of by-products, measures are taken to prevent nitrobenzene from breaking through in the hydrogenation reactor, wherein the measures which are taken to prevent nitrobenzene from breaking through in the hydrogenation reactor comprise at least one of:
- scheduling regeneration or replacement of the catalyst and foresee sufficient inventory of pure aniline;
- decreasing plant load to postpone catalyst deactivation;
- temporarily increase the time intervals for analysing the composition of the crude reaction product;
- increasing the temperature of the reaction to increase reactivity and to postpone the catalyst deactivation.

2. The process according to claim 1, wherein the reaction in (a) is carried out in the gas phase or in the liquid phase.

3. The process according to claim 1 or 2, wherein the reaction in (a) is carried out in the gas phase and the crude reaction product is at least partly condensed before starting working-up in (b).

4. The process according to claim 3, wherein the composition of the at least partly condensed crude reaction product is analysed.

5. The process according to claim 3 or 4, wherein the at least partially condensing of the crude reaction product is carried out in at least two condensation steps.

6. The process according to claim 5, wherein the composition of the partly condensed crude reaction product obtained in the first condensation step is analysed.

7. The process according to any of claims 1 to 6, wherein the measures to prevent nitrobenzene from breaking through in the hydrogenation reactor are taken when the amount of by-products in the crude reaction product increases more than 10 % compared to the concentration during normal operation.

8. The process according to any of claims 1 to 7, wherein analysing the composition of the crude reaction product is carried out by online analysis processes.

9. The process according to any of claims 1 to 7, wherein for analysing the composition of the crude reaction product a sample is taken and the sample is analysed.

10. The process according to any of claims 1 to 9, wherein the composition of the crude reaction product is analysed in regular time intervals which are shorter than 24 h.

11. The process according to any of claims 1 to 10, wherein the by-product, the proportion of which is analysed, is at least one of aminophenols, n-phenyl-1,2-benzenediamine, n-phenyl-1,4-benzenediamine, phenazine, phenol, nitrobenzene, and unidentifiable high boiling components.

12. The process according to any of claims 1 to 11, wherein analysing of the composition of the crude reaction product is carried out by chromatographic or spectroscopic techniques.

## Patentansprüche

1. Ein Verfahren zur Herstellung von Anilin durch katalytische Hydrierung von Nitrobenzol, bestehend:
(a) die Reaktion von Wasserstoff und Nitrobenzol in Anwesenheit eines Katalysators in einem Hydrierungsreaktor, wodurch ein rohes Reaktionsprodukt entsteht;
(b) das rohe Reaktionsprodukt zu gewinnen, um reines Anilin zu gewinnen,
wobei die Zusammensetzung des Rohreaktionsprodukts kontinuierlich oder in regelmäßigen Abständen vor Beginn der Verarbeitung in (b) analysiert wird, um den Anteil der Nebenprodukte zu bestimmen, wobei im Falle einer Zunahme der Nebenprodukte Maßnahmen ergriffen werden, um das Durchbrechen von Nitrobenzol im Hydrierungsreaktor zu verhindern, wobei die Maßnahmen zur Verhinderung des Durchbruchs von Nitrobenzol im Hydrierungsreaktor mindestens eine von den folgenden umfassen:
- Planung der Regeneration oder Austausch des Katalysators und Vorherstellung eines ausreichenden Inventars an reinem Anilin;
- Verringerung der Anlagenlast, um die Deaktivierung des Katalysators hinauszuzögern;
- die Zeitintervalle für die Analyse der Zusammensetzung des Rohölprodukts vorübergehend zu erhöhen;
- Erhöhung der Temperatur der Reaktion, um die Reaktivität zu erhöhen und die Deaktivierung des Katalysators hinauszuzögern.

2. Der Prozess gemäß Anspruch 1, bei dem die Reaktion in (a) in der Gasphase oder in der flüssigen Phase durchgeführt wird.

3. Der Prozess gemäß Anspruch 1 oder 2, bei dem die Reaktion in (a) in der Gasphase durchgeführt wird und das Rohprodukt der Reaktion zumindest teilweise kondensiert wird, bevor in (b) mit dem Aufbau begonnen wird.

4. Der Prozess gemäß Anspruch 3, bei dem die Zusammensetzung des zumindest teilweise verdichteten Rohreaktionsprodukts analysiert wird.

5. Der Prozess gemäß Behauptung 3 oder 4, bei dem die zumindest teilweise Kondensation des Rohreaktionsprodukts in mindestens zwei Kondensationsschritten durchgeführt wird.

6. Der Prozess gemäß Behauptung 5, bei dem die Zusammensetzung des teilweise kondensierten Rohreaktionsprodukts, das im ersten Kondensationsschritt gewonnen wurde, analysiert wird.

7. Der Prozess gemäß einer der Ansprüche 1 bis 6, bei dem Maßnahmen zur Verhinderung des Durchbruchs von Nitroben-Zene im Hydrierungsreaktor ergriffen werden, wenn die Menge von
Die Nebenprodukte im rohem Reaktionsprodukt steigen im Normalbetrieb im Vergleich zur Konzentrierung um mehr als 10 % an.

8. Der Prozess gemäß einem der Ansprüche 1 bis 7, bei dem die Zusammensetzung des Rohreaktionsprodukts analysiert wird, erfolgt durch Online-Analyseverfahren.

9. Der Prozess gemäß einem der Ansprüche 1 bis 7, bei dem zur Analyse der Zusammensetzung des Rohreaktionsprodukts eine Probe entnommen und die Probe analysiert wird.

10. Der Prozess gemäß jedem der Ansprüche 1 bis 9, bei dem die Zusammensetzung des Rohprodukts in regelmäßigen Zeitintervallen analysiert wird, die kürzer als 24 Stunden sind.

11. Der Prozess gemäß einer der Ansprüche 1 bis 10, bei dem das Nebenprodukt, dessen Anteil analysiert wird, mindestens eines von Aminophenolen, n-phenyl-1,2-benzendiamin, n-phe-nyl-1,4-benzendiamin, Phenazin, Phenol, Nitrobenzol und nicht identifizierbaren hochsiedenden Bestandteilen ist.

12. Der Prozess gemäß einem der Ansprüche 1 bis 11, bei dem die Analyse der Zusammensetzung des Rohreaktionsprodukts mittels chromatographischer oder spektroskopischer Techniken durchgeführt wird.

## Revendications

1. Un procédé de production d'aniline par hydrogénation catalytique du nitrobenzène comprenant :
(a) réaction de l'hydrogène et du nitrobenzène en présence d'un catalyseur dans un réacteur d'hydrogénation, formant un produit de réaction brut ;
(b) Travailler le produit de réaction brut pour obtenir de l'aniline pure,
où la composition du produit de la réaction brute est analysée en continu ou à intervalles de temps Regu-Lar avant de commencer le travail en (b), afin de déterminer la proportion de sous-produits, dans le cas, en cas d'augmentation de la quantité de sous-produits, des mesures sont prises pour empêcher le nitrobenzène de percer dans le réacteur d'hydrogénation, les mesures prises pour empêcher le nitrobenzène de percer dans le réacteur d'hydrogénation comprennent au moins une des suivantes :
- la planification de la régénération ou du remplacement du catalyseur et la prévision d'un stock suffisant d'aniline pure ;
- diminution de la charge de l'usine pour retarder la désactivation du catalyseur ;
- augmenter temporairement les intervalles de temps pour analyser la composition du produit de réaction brut ;
- augmenter la température de la réaction pour accroître la réactivité et retarder la désactivation du catalyseur.

2. Le procédé selon l'affirmation 1, dans lequel la réaction en (a) est réalisée en phase gazeuse ou en phase liquide.

3. Le procédé, selon la revendication 1 ou 2, dans lequel la réaction dans (a) est réalisée en phase gazeuse et le produit brut de la réaction est au moins partiellement condensé avant de commencer à être travaillé en (b).

4. Le procédé selon l'affirmation 3, dans lequel la composition du produit de réaction brute au moins partiellement densifié est analysée.

5. Le procédé, selon la revendication 3 ou 4, consiste à condenser au moins partiellement le produit de réaction brut en au moins deux étapes de condensation.

6. Le procédé selon la revendication 5, dans lequel la composition du produit de réaction brut partiellement condensé obtenu lors de la première étape de condensation est analysée.

7. Le procédé selon l'une des revendications 1 à 6, dans lequel les mesures pour empêcher que le nitrobène-zène ne perde dans le réacteur d'hydrogénation sont prises lorsque la quantité de
Les sous-produits dans le produit de réaction brut augmentent de plus de 10 % par rapport à la con-centration lors du fonctionnement normal.

8. Le processus selon l'une des revendications 1 à 7, dans lequel l'analyse de la composition du produit réactionnel brut est réalisée par des processus d'analyse en ligne.

9. Le processus selon l'une des revendications 1 à 7, dans lequel pour analyser la composition du produit de réaction brut est prélevé un échantillon et l'échantillon est analysé.

10. Le processus selon l'une des revendications 1 à 9, où la composition du produit de réaction brute est analysée à des intervalles réguliers inférieurs à 24 heures.

11. Le procédé selon l'une des revendications 1 à 10, dans lequel le sous-produit, dont la proportion est analysée, est au moins un composé d'aminophénols, de n-phényl-1,2-benzénadiamine, de n-phé-nyl-1,4-benzénadiamine, de phénazine, de phénol, de nitrobenzène et de composants à forte ébullition non identifiables.

12. Le procédé selon l'une des revendications 1 à 11, au cours duquel l'analyse de la composition du produit de réaction brut est réalisée par des techniques chromatographiques ou spectroscopiques.
